# EUROPEAN PATENT APPLICATION

(11) **EP 4 678 119 A1**
(43) Date of publication of application: **14.01.2026**
(21) Application number: 24187820.6
(22) Date of filing: 10.07.2024
(51) Int. Cl.: A61B 17/00, A61B 90/00

(54) **LARGE BORE VASCULAR CLOSURE SYSTEM**

(71) Applicant: SpioRAD Medical Limited, Dublin D13 Y5Y0 (IE)
(72) Inventor: O'MALLEY, Judi, Dublin, D13 Y5Y0 (IE); ARNOUS, Samer, Limerick, V94 E6X4 (IE); O'ROURKE, John, Tipperary, E91 ED65 (IE); MANNING, Daragh Michael, Dublin, D03 RX06 (IE); BRUGGEMANN, Martin, Laois, R32 N6W8 (IE); MOLLOY, Patrick, Clare, V97 A079 (IE); KEANE, Sarah, Kildare, W12 VE89 (IE); WOMBWELL, Thomas, Surrey, TW1 1HB (GB)
(74) Representative: Purdylucey Intellectual Property

(57) **Abstract**

A large bore extra-vascular closure system to occlude a large bore tissue tract proximal to a blood vessel comprising an introducer device (190) having a handle (200) housing an ejector module (210) provided with a delivery conduit (240) extending distally from the handle (200) for delivering an occlusion device (10) to a tissue tract, and an occlusion device deployment module (220) provided with a deployment tube (221) extending distally from the handle (200) within the delivery conduit (240) for deploying the occlusion device (10); a bioresorbable occlusion device (10) configured for adjustment by the deployment module (220) between an elongate delivery configuration in which the occlusion device (10) is dimensioned to fit within the delivery conduit (240) and a squat deployed configuration in which the occlusion device (10) is expanded radially outwardly; a control wire operably connected to the occlusion device (10), and an injectable filler material configured for injection into the occlusion device (10) in the squat deployed configuration wherein the handle (200) comprises a control wire locking module (230) configured to hold the occlusion device (10) in the squat deployed configuration.

## Description

### Field of the Invention

This invention relates to a large bore vascular closure system.

### Background to the Invention

In percutaneous medical procedures, an opening may be created in a wall of a blood vessel to allow for the insertion of various medical devices which can be navigated through the blood vessel to a site to be treated. For example, after initial access into the blood vessel is obtained, a medical device may be inserted through the tissue tract created between the skin, or epidermis, of the patient down through the subcutaneous tissue and into the opening formed in the blood vessel. The medical device may then be navigated through the blood vessel to the treatment site.

Once the procedure is completed, the medical device(s) or other equipment introduced into the blood vessel may be retracted from the body through the blood vessel, out the opening in the wall of the blood vessel, and out through the tissue tract. The physician or other medical technician is presented with the challenge of trying to close the opening in the blood vessel and/or the tissue tract formed in the epidermis and subcutaneous tissue. Several different device structures, assemblies, and methods are known for closing the opening in the blood vessel and/or tissue tract, each having certain advantages and disadvantages. There is an ongoing need to provide alternative medical devices as well as alternative methods for manufacturing and/or using medical devices.

Current closure devices on the market do not extend to be inclusive of full-size requirement. Also, complication rates are high (major and minor ranging from 12% to 29%) with problematic usability known, often resulting in numerous devices being used per procedure. Issues of haemostasis results in blood loss, and risks of embolization from intravascular devices result in acute limb ischemia risk limb loss often resulting in immediate surgical escalation and intervention.

A known closure system can be made up of an occlusion device for closing blood vessel openings and an introducer device for delivering the occlusion device to the openings and actuating the occlusion device in the opening via control wires in the form of sutures extending between the occlusion device and the introducer device. For example, PCT Patent Specification No. WO 2023/072972 A1 describes such a closure system in which an occlusion device is movable between delivery and squat radially expanded deployed configurations using control wires subjected to a pulling force and braking/anchor elements on the control wires. However, the need for control wires provided with braking and anchor elements to move the occlusion device into the squat radially expanded deployed configuration complicates the construction, assembly and operation of the closure system.

It is an object of the invention to overcome at least one of the above-referenced problems.

### Summary of the Invention

The Applicant has addressed the problems of the prior art by providing a large bore closure system that is fully extravascular, obviating the risks of embolization whilst being simple and effective in construction. This is achieved by providing an occlusion device configured for deployment in a tissue tract proximal of an opening in a blood vessel such that upon deployment no part of the device is disposed in the blood vessel.

The occlusion device generally comprises a waist section that is configured to expand radially outwardly when the device is compressed axially by the introducer device. Due to the radial expansion feature, the device is adaptable for use with tissue tracts of varying bore size, for example from 12Fr to 26Fr (where Fr is French scale) or greater. Thus, the large bore vascular closure system of the invention provides decreased risk by not having anything within the artery to cause embolization, furthermore the occlusion device can be deployed with ease using the introducer device to close tissue tracts generated by the largest percutaneous access devices on the market without requiring the use of complex control wire constructions. More particularly, due to the use of a deployment module that exerts a pushing force on the occlusion device to compress the occlusion device in to the squat deployed configuration and optionally also a control wire locking module co-operable with the deployment module to temporarily hold occlusion devices in the deployed configuration until injected with a filler material to fix the deployed configuration, control wires provided with anchors, brakes and the like are not required.

The introducer device can comprise a control wire (e.g. a suture) operated occlusion device deployment system configured to deliver the occlusion device into the tissue tract comprising a handle having an ejector module for ejecting an occlusion device from the introducer device, a deployment module to move the occlusion device between an elongate delivery position and a squat radially expanded deployment position and a control wire locking module connected with the deployment module via the control wire configured to (temporarily) maintain/hold the occlusion device in the deployed squat and radially expanded configuration during deployment of the occlusion device in the tissue tract. The introducer device generally comprises a through lumen for receipt of a guidewire and to allow delivery of an injectable filler material into a lumen of the deployed occlusion device to permanently maintain the occlusion device in a squat radially expanded configuration.

The ejector module is actuatable to expose the occlusion device proud of a distal end of the introducer device. The control wire may be attached under tension between a distal end of the occlusion device and a control wire locking module whereby an axial compressive pushing force applied by the deployment module to the occlusion device in the elongate delivery position against the tension urges the proximal end of the occlusion device towards the distal end of the occlusion device to deploy the occlusion device into the squat deployed configuration. The control wire may extend into the occlusion device through an aperture in the proximal end of the occlusion device, loop through apertures in the distal end, and extend out of the occlusion device through a second aperture in the proximal end of the occlusion device and be tensioned by clamping the control wire in the control wire locking module

As indicated above, the control wire locking module is configured to be co-operable with the control wire upon actuation of the deployment module so that the axial compressive pushing force of the deployment module opposes the tension on the control wire to urge the proximal end of the occlusion device towards the distal end of the occlusion device in a controlled manner to temporarily maintain/hold the occlusion device in the deployed squat and radially expanded configuration in a tissue tract during deployment. The injectable filler material is typically a bioresorbable injectable filler material such as a thermosetting material or an adhesive and is injectable into the through lumen of the introducer device when the occlusion device is in the temporary squat and radially expanded configuration to occlude the occlusion device lumen and permanently maintain/hold (i.e. until bioresorption occurs) the occlusion device in the deployed configuration when set. The control wire locking module can then be operated to release the control wire and disconnect the introducer device from the occlusion device.

According to the invention there is provided a large bore extra-vascular closure system to occlude a large bore tissue tract proximal to a blood vessel, comprising:
an introducer device having
   a handle housing an ejector module provided with a delivery conduit extending distally from the handle for delivering an occlusion device to a tissue tract,
   an occlusion device deployment module provided with a deployment tube extending distally from the handle within the delivery conduit for deploying the occlusion device, and
a bioresorbable occlusion device having a distal end and a proximal end configured for adjustment by the deployment module between an elongate delivery configuration in which the occlusion device is dimensioned to fit within the delivery conduit and a squat deployed configuration in which the occlusion device is expanded radially outwardly,
wherein the deployment tube is configured upon actuation to push the proximal end of the occlusion device towards the distal end of the occlusion device to expand the occlusion device radially outwardly into the squat deployed configuration.

In any embodiment, the introducer device comprises a control wire operably connecting the distal end of the occlusion device to a control wire locking module co-operable with the deployment module to hold the occlusion device in the squat deployed configuration and an injectable filler material configured for injection into the occlusion device in the squat deployed configuration.

In any embodiment, the introducer device comprises a through lumen extending through the handle and the delivery conduit configured to receive a guidewire and to allow delivery of the injectable filler material into a deployed occlusion device to permanently maintain the occlusion device in a squat radially expanded configuration.

In any embodiment, the control wire locking module comprises a lock and a key complementary with the lock in which the key is movable between a control wire holding position in which the occlusion device is held in the squat deployed configuration and a control wire release position.

In any embodiment, the lock comprises a keyway for receiving the key and the key comprises a key shaft with a biting for holding the control wire between the biting and the keyway in the control wire holding position.

In any embodiment, the biting is configured to define an interference fit with the keyway in the control wire holding position.

In any embodiment, the locking module is mounted in the handle between the ejector module and the deployment module.

In any embodiment, the locking module comprises a control wire guide channel and/or a deployment tube guide channel configured to support the deployment tube in the handle.

In any embodiment, the ejector module comprises a slidable ejector carriage slidably mounted in the handle and an elongate ejector sheath attached to the ejector carriage defining the delivery conduit, the ejector carriage being slidable between an occlusion device delivery configuration in which the ejector sheath is in an extended position over the occlusion device and a retracted occlusion device deployment position in which the occlusion device is exposed for deployment by the deployment module.

In any embodiment, the ejector carriage comprises a housing slidable in a carriage track in the handle).

In any embodiment, the housing comprises a bore for receiving the deployment tube.

In any embodiment, the ejector module comprises a push-button mounted on the ejector carriage to effect movement of the ejector carriage between the occlusion device delivery configuration and the occlusion device deployment position.

In any embodiment, the ejector module comprises an ejector module catch towards the end of the carriage track to hold the ejector module in the occlusion device delivery configuration and the occlusion device deployment position.

In any embodiment, the deployment module comprises a rotation to translation movement actuator.

In any embodiment, the rotation to translation movement actuator comprises a translatable rod in the handle connected with a rotor towards a proximal end of the handle).

In any embodiment, the translatable rod comprises a tubular shaft having laterally extending male fingers and the rotor comprises an internally threaded body configured to receive the male fingers of the translating rod in a rotating relationship.

In any embodiment, the body comprises two oppositely disposed threads configured to receive two respective male fingers and to limit rotation of the rotor to about 180° between an occlusion device elongate delivery position and an occlusion device squat radially expanded deployment position.

In any embodiment, the through lumen of the introducer device has a diameter of at least 0.5 mm, for example 0.5 to 2.0 mm or 0.5 to 1.5 mm.

In any embodiment, the control wire is a suture, for example a bioresorbable suture.

In any embodiment, the occlusion device and/or the control wire are bioresorbable.

In any embodiment, the system comprises a plurality of control wires connecting the deployment module and the occlusion device.

In any embodiment the control wires are connected to the occlusion device in a symmetrical manner (e.g., connected to opposite sides) to balance the deployment of the device.

In any embodiment, the control wire is a closed loop.

In any embodiment, the control wire has free ends.

In any embodiment, the system is configured such that rotation of the rotor of the deployment module in a first direction causes the occlusion device to deploy into the squat radially expanded configuration and rotation of the rotor in a second direction causes the occlusion device to return to the elongated delivery configuration.

In any embodiment, the rotor comprises a graduated scale indicating a range of large bore sizes.

In any embodiment, the system comprises a syringe device comprising a delivery conduit configured to extend through the through lumen of the introducer device to deliver the injectable filler material into the bioresorbable occlusion device when the bioresorbable occlusion device is deployed.

In any embodiment, the system comprises a guidewire in which the introducer device is configured to be mounted on the guidewire.

In any embodiment, the occlusion device has a length of 4.0 to 12.0 mm in a delivery configuration.

In any embodiment, the occlusion device has a length of 0.5 to 4.0 mm in a deployed configuration.

In any embodiment, the occlusion device has a maximum diameter of 4.0 to 8.7 mm in a delivery configuration.

In any embodiment, the occlusion device has a maximum diameter of 8.0 to 8.7 mm in a deployed configuration.

In a further aspect, the invention also relates to a large bore extra-vascular closure system to occlude a large bore tissue tract proximal to a blood vessel, comprising:
an introducer device comprising
   a handle housing an ejector module provided with a delivery conduit extending distally from the handle for delivering an occlusion device to a tissue tract, and an occlusion device deployment module provided with a deployment tube extending distally from the handle within the delivery conduit for deploying the occlusion device, and
a bioresorbable occlusion device having a distal end and a proximal end configured for adjustment by the deployment module between an elongate delivery configuration in which the occlusion device is dimensioned to fit within the delivery conduit and a squat deployed configuration in which the occlusion device is expanded radially outwardly,
wherein the ejector module comprises a slidable ejector carriage slidably mounted in the handle and an elongate ejector sheath attached to the ejector carriage defining the delivery conduit, the ejector carriage being slidable between an occlusion device delivery configuration in which the ejector sheath is in an extended position over the occlusion device and a retracted occlusion device deployment position in which the occlusion device is exposed for deployment by the deployment module.

In any embodiment, the introducer device comprises a control wire operably connecting the distal end of the occlusion device to a control wire locking module co-operable with the deployment module to hold the occlusion device in the squat deployed configuration and an injectable filler material configured for injection into the occlusion device in the squat deployed configuration.

In any embodiment, the ejector carriage comprises a housing slidable in a carriage track in the handle.

In any embodiment, the housing comprises a bore for receiving the deployment tube.

In any embodiment, the ejector module comprises a push-button mounted on the ejector carriage to effect movement of the ejector carriage between the occlusion device delivery configuration and the occlusion device deployment position.

In any embodiment, the ejector module comprises an ejector module catch towards the end of the carriage track to hold the ejector module in the occlusion device delivery configuration and the occlusion device deployment position.

In any embodiment, the handle comprises a control wire locking module configured to hold the occlusion device in the squat deployed configuration.

In any embodiment, the introducer device comprises a through lumen extending through the handle and the delivery conduit configured to receive a guidewire and to allow delivery of the injectable filler material into a deployed occlusion device to permanently maintain the occlusion device in a squat radially expanded configuration.

In any embodiment, the control wire locking module comprises a lock and a key complementary with the lock in which the key is movable between a control wire holding position in which the occlusion device is held in the squat deployed configuration and a control wire release position.

In any embodiment, the lock comprises a keyway for receiving the key and the key comprises a key shaft with a biting for holding the control wire between the biting and the keyway in the control wire holding position.

In any embodiment, the biting is configured to define an interference fit with the keyway in the control wire holding position.

In any embodiment, the locking module is mounted in the handle between the ejector module and the deployment module.

In any embodiment, the locking module comprises a control wire guide channel configured to guide a control wire between the occlusion device and the deployment module and/or a deployment tube guide channel configured to support the deployment tube in the handle.

In any embodiment, the deployment module comprises a rotation to translation movement actuator.

In any embodiment, the rotation to translation movement actuator comprises a translatable rod in the handle connected with a rotor towards a proximal end of the handle).

In any embodiment, the translatable rod comprises a tubular shaft having laterally extending male fingers and the rotor comprises an internally threaded body configured to receive the male fingers of the translating rod in a rotating relationship.

In any embodiment, the body comprises two oppositely disposed threads configured to receive two respective male fingers and to limit rotation of the rotor to about 180° between an occlusion device elongate delivery position and an occlusion device squat radially expanded deployment position.

In yet a further aspect, the invention relates to a large bore extra-vascular closure system to occlude a large bore tissue tract proximal to a blood vessel, comprising:
an introducer device comprising
   a handle housing an ejector module provided with a delivery conduit extending distally from the handle for delivering an occlusion device to a tissue tract, and an occlusion device deployment module provided with a deployment tube extending distally from the handle within the delivery conduit for deploying the occlusion device, and
a bioresorbable occlusion device configured for adjustment by the deployment module between an elongate delivery configuration in which the occlusion device is dimensioned to fit within the delivery conduit and a squat deployed configuration in which the occlusion device is expanded radially outwardly;
wherein the deployment module comprises a rotation to translation movement actuator.

In any embodiment, the introducer device comprises a control wire operably connecting the distal end of the occlusion device to a control wire locking module co-operable with the deployment module to hold the occlusion device in the squat deployed configuration and an injectable filler material configured for injection into the occlusion device in the squat deployed configuration.

In any embodiment, the rotation to translation movement actuator comprises a translatable rod in the handle connected with a rotor towards a proximal end of the handle).

In any embodiment, the translatable rod comprises a tubular shaft having laterally extending male fingers and the rotor comprises an internally threaded body configured to receive the male fingers of the translating rod in a rotating relationship.

In any embodiment, the body comprises two oppositely disposed threads configured to receive two respective male fingers and to limit rotation of the rotor to about 180° between an occlusion device elongate delivery position and an occlusion device squat radially expanded deployment position.

In any embodiment, the introducer device comprises a through lumen extending through the handle and the delivery conduit configured to receive a guidewire and to allow delivery of the injectable filler material into a deployed occlusion device to permanently maintain the occlusion device in a squat radially expanded configuration.

In any embodiment, the control wire locking module comprises a lock and a key complementary with the lock in which the key is movable between a control wire holding position in which the occlusion device is held in the squat deployed configuration and a control wire release position.

In any embodiment, the lock comprises a keyway for receiving the key and the key comprises a key shaft with a biting for holding the control wire between the biting and the keyway in the control wire holding position.

In any embodiment, the biting is configured to define an interference fit with the keyway in the control wire holding position.

In any embodiment, the locking module is mounted in the handle between the ejector module and the deployment module.

In any embodiment, the locking module comprises a control wire guide channel and/or a deployment tube guide channel configured to support the deployment tube in the handle.

In any embodiment, the ejector module comprises a slidable ejector carriage slidably mounted in the handle and an elongate ejector sheath attached to the ejector carriage defining the delivery conduit, the ejector carriage being slidable between an occlusion device delivery configuration in which the ejector sheath is in an extended position over the occlusion device and a retracted occlusion device deployment position in which the occlusion device is exposed for deployment by the deployment module.

In any embodiment, the ejector carriage comprises a housing slidable in a carriage track in the handle).

In any embodiment, the housing comprises a bore for receiving the deployment tube.

In any embodiment, the ejector module comprises a push-button mounted on the ejector carriage to effect movement of the ejector carriage between the occlusion device delivery configuration and the occlusion device deployment position.

In any embodiment, the ejector module comprises an ejector module catch towards the end of the carriage track to hold the ejector module in the occlusion device delivery configuration and the occlusion device deployment position.

In another aspect, the invention relates to a large bore extra-vascular closure system to occlude a large bore tissue tract proximal to a blood vessel, comprising:
an introducer device comprising
   a handle housing an ejector module provided with a delivery conduit extending distally from the handle for delivering an occlusion device to a tissue tract, and an occlusion device deployment module provided with a deployment tube extending distally from the handle within the delivery conduit for deploying the occlusion device;
a bioresorbable occlusion device configured for adjustment by the deployment module between an elongate delivery configuration in which the occlusion device is dimensioned to fit within the delivery conduit and a squat deployed configuration in which the occlusion device is expanded radially outwardly;
a control wire operably connecting the occlusion device to a control wire locking module co-operable with the deployment module to hold the occlusion device in the squat deployed configuration, and
an injectable filler material configured for injection into the occlusion device in the squat deployed configuration,

In any embodiment, the introducer device comprises a through lumen extending through the handle and the delivery conduit configured to receive a guidewire and to allow delivery of the injectable filler material into a deployed occlusion device to permanently maintain the occlusion device in a squat radially expanded configuration.

In any embodiment, the control wire locking module comprises a lock and a key complementary with the lock in which the key is movable between a control wire holding position in which the occlusion device is held in the squat deployed configuration and a control wire release position.

In any embodiment, the lock comprises a keyway for receiving the key and the key comprises a key shaft with a biting for holding the control wire between the biting and the keyway in the control wire holding position.

In any embodiment, the biting is configured to define an interference fit with the keyway in the control wire holding position.

In any embodiment, the locking module is mounted in the handle between the ejector module and the deployment module.

In any embodiment, the locking module comprises a control wire guide channel and/or a deployment tube guide channel configured to support the deployment tube in the handle.

In any embodiment, the ejector module comprises a slidable ejector carriage slidably mounted in the handle and an elongate ejector sheath attached to the ejector carriage defining the delivery conduit, the ejector carriage being slidable between an occlusion device delivery configuration in which the ejector sheath is in an extended position over the occlusion device and a retracted occlusion device deployment position in which the occlusion device is exposed for deployment by the deployment module.

In any embodiment, the ejector carriage comprises a housing slidable in a carriage track in the handle).

In any embodiment, the housing comprises a bore for receiving the deployment tube.

In any embodiment, the ejector module comprises a push-button mounted on the ejector carriage to effect movement of the ejector carriage between the occlusion device delivery configuration and the occlusion device deployment position.

In any embodiment, the ejector module comprises an ejector module catch towards the end of the carriage track to hold the ejector module in the occlusion device delivery configuration and the occlusion device deployment position.

In any embodiment, the deployment module comprises a rotation to translation movement actuator.

In any embodiment, the rotation to translation movement actuator comprises a translatable rod in the handle connected with a rotor towards a proximal end of the handle).

In any embodiment, the translatable rod comprises a tubular shaft having laterally extending male fingers and the rotor comprises an internally threaded body configured to receive the male fingers of the translating rod in a rotating relationship.

In any embodiment, the body comprises two oppositely disposed threads configured to receive two respective male fingers and to limit rotation of the rotor to about 180° between an occlusion device elongate delivery position and an occlusion device squat radially expanded deployment position.

In another aspect, the invention provides a method of closing a large bore tissue tract in a subject comprising:
providing a system according to the invention;
advancing a distal end of the introducer device into a large bore tissue tract in the subject over a guidewire to a position proximal of a blood vessel;
actuating the ejector module to eject the occlusion device into the large bore tissue tract proximally of an artery or vein;
actuating the locking module to clamp and tension the control wires
deploying the occlusion device with the deployment module by rotating the rotor to axially translate or push the deployment tube distally to compress the occlusion device into the occlusion device squat radially expanded deployment position;
injecting the injectable filler material into the occlusion device to permanently fix the occlusion device in the squat radially expanded deployment position, and,
unlocking the locking module to unclamp the control wires.

In any embodiment, the occlusion device is ejected by retracting an ejector sheath of the ejector module to expose the occlusion device.

In any embodiment, rotation of the rotor in a first direction causes the occlusion device to deploy into the squat radially expanded configuration and rotation of the rotor in a second direction causes the occlusion device to return to the elongated delivery configuration.

In any embodiment, the introducer device comprises a through lumen extending through the handle and the delivery conduit configured to receive the guidewire and to allow delivery of the injectable filler material into a deployed occlusion device to permanently maintain the occlusion device in the squat radially expanded configuration.

In any embodiment, the method is for closing a tissue tract in fluid communication with an opening in a blood vessel in a subject.

In any embodiment, the method is for closing a tissue tract in fluid communication with an opening in a femoral artery in a subject.

In any embodiment, the occlusion device is deployed 0.5-2.0 cm distally of the opening in the blood vessel.

In any embodiment, the position of the occlusion device is monitored using imaging, typically contrast imaging.

In any embodiment, the occlusion device is deployed extra-vascularly, typically just proximally of the adventitial layer of an artery or vein.

In any embodiment, the occlusion device is radially expanded to a diameter that is greater than a diameter of the tissue tract, typically at least 5%, 10%, 15% or 20% greater.

In any embodiment, occlusion of the tissue tract is monitored using imaging, typically contrast imaging.

In any embodiment, the guidewire is removed leaving the occlusion device in-situ in the tissue tract.

In any embodiment, the introducer device comprises a control wire release mechanism configured upon actuation to uncouple the control wires from the introducer device (e.g., from the actuator of the introducer device) to allow the occlusion device and introducer device to be uncoupled and the introducer device to be retracted and removed.

Other aspects and preferred embodiments of the invention are defined and described in the other claims set out below.

### Brief Description of the Figures

**Figure 1** is a side elevational view of one example of an occlusion device suitable for forming part of a large bore extra-vascular closure system of the invention made up of an inner frame and an outer fluid impermeable sheath, shown in an elongated delivery configuration;
**Figure 2** is a side elevational view of the occlusion device of Figure 1, shown in a squat deployed configuration;
**Figure 3** is a perspective view from above and one side of the inner frame of Figure 1 separated from the outer sheath, shown in an elongated delivery configuration;
**Figure 4** is a perspective view from above and one side of the outer flexible fluid impermeable sheath separated from the inner frame of Figure 1, shown in an elongated delivery configuration;
**Figure 5** is a perspective view from above and one side of the frame of Figure 3, shown in a squat configuration;
**Figure 6** is a perspective view from above and one side of the outer flexible fluid impermeable sheath of Figure 4, shown in a squat deployed configuration;
**Figure 7** is a perspective view from above and one side of a balloon dimensioned to fit within the frame to receive and contain the injectable filler material such as collagen to permanently maintain the occlusion device in the deployed squat and radially expanded configuration in-vivo;
**Figure 8** is a perspective view from above and one side of an introducer device also forming part of the large bore extra-vascular closure system of the invention generally made up of an occlusion device ejector module (with its retractable tubular distal ejector sheath omitted for clarity), a control wire locking module and a deployment module at a handle to effect deployment of the occlusion device into the squat radially expanded configuration with the ejector module in the ejector sheath retracted position;
**Figure 9** is a longitudinal cross-sectional view through the introducer device of Figure 8 with the retractable distal ejector sheath in place over the deployment tube and defining a delivery conduit and with the ejector module in the occlusion device delivery configuration (i.e. the ejector sheath is in an extended position) to define a protective delivery chamber in the delivery conduit for an occlusion device with an occlusion device contained within the delivery chamber;
**Figure 10** is a longitudinal cross-sectional view through the device of Figure 9 with the ejector module in the retracted deployment position to expose the occlusion device for deployment;
**Figure 11** is an enlarged partially exploded and cross-sectional view through the handle of the introducer device with the ejector module made up of a slidable carriage and a push-button separated from the handle and the ejector sheath, locking module and deployment module omitted for clarity;
**Figure 12** is a further enlarged perspective view from above and one side of the slidable carriage and push-button of the ejector module of Figure 11 with the push-button detached from the slidable carriage;
**Figure 13** is an enlarged partially exploded and cross-sectional view through the handle of the introducer device of Figure 11 with the ejector module in place in the handle in the occlusion device delivery configuration and the control wire locking module detached from the handle for clarity;
**Figure 14** is a further enlarged perspective view from above and one side of the control wire locking module with the key separated from the lock of the locking module;
**Figure 15** is an enlarged partially exploded and cross-sectional perspective view through the handle of the introducer device of Figure 13 with the ejector module and the control wire locking module in place in the handle with a right hand side of the internally threaded knob-like rotor of the rotation to translation movement actuator of the deployment module detached from the handle for clarity and the translating rod of the rotation to translation movement actuator mounted in the handle;
**Figure 16** is a further enlarged cross-sectional perspective view of the proximal end of the handle of Figure 15 with the right hand side of the rotor mounted on the handle to rotatably engage the rod with the male lugs of the rod inserted between the groove of the female thread of the rotor;
**Figure 17** is a cross-sectional perspective view through the introducer device of the invention with the occlusion device ejector module (with its retractable tubular ejector sheath omitted for clarity), the control wire locking module and the deployment module mounted in the right hand side of the handle and the internally threaded knob-like rotor;
**Figure 18** is a perspective view from above and the proximal end of the handle of the introducer device with rotors omitted and the translating rod mounted in the neck of the handle at the proximal end;
**Figure 19** is an enlarged perspective view from above and the proximal end of the handle of Figure 18 with biased control spring of the deployment module separated from the handle;
**Figure 20** is an enlarged side view in partial cross-section of the occlusion device of

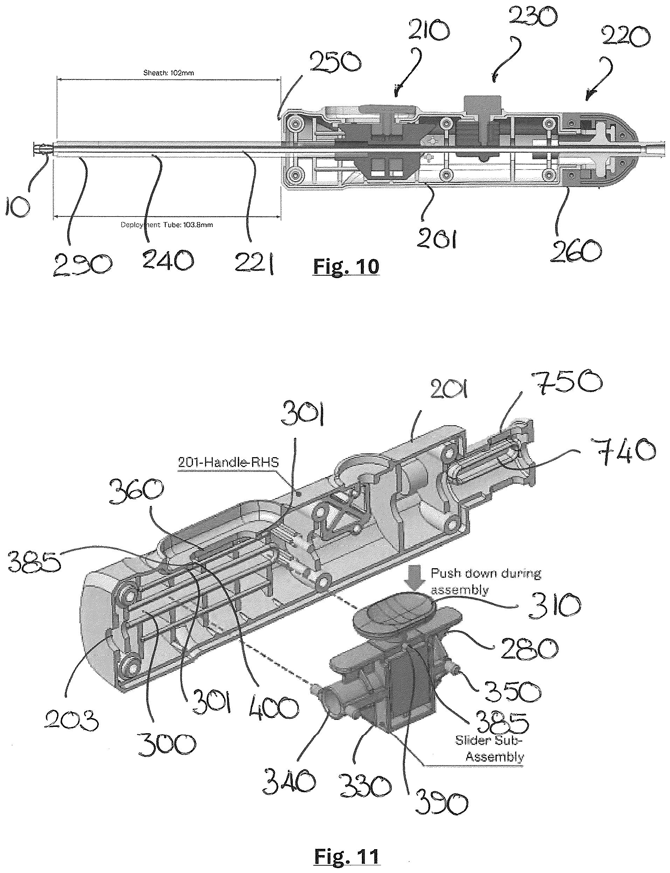

Figure 10 ejected by the ejector module from the delivery conduit still in the elongate delivery configuration with the axial direction of movement of the deployment tube of the deployment module indicated by the arrows, and

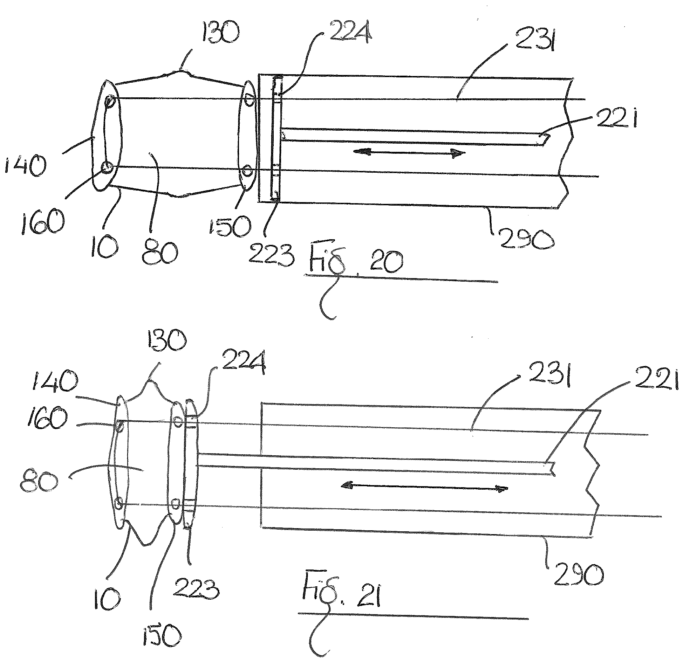

Figure 21 is a side view in partial cross-section of the occlusion device of Figure 20 with the occlusion device axially compressed by the deployment tube into the squat deployed configuration.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g., the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g., the administration of an agent to a subject) which prevents or delays the onset or progression of a disease or reduces (or eradicates) its incidence within a treated population. In this case, the term treatment is used synonymously with the term "prophylaxis".

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

As used herein, the term "tissue tract" refers to a bore in tissue providing fluid communication from a body surface to an opening in a blood vessel.

As used herein, the term "large bore" refers to bore having a French size of 12F (4.0mm/0.158 inches) to 26F (8.7mm/0.341 inches) or greater.

As used herein, the term "extra-vascular" as applied to the closure device refers to a closure device configured to implantation in a tissue tract proximal of a blood vessel.

As used herein, the term "occlusion device" refers to any device that is radially expansible from a delivery configuration to a radially expanded deployed configuration to occlude a lumen such as a tissue tract. The occlusion device can be fluid impermeable during deployment. In one form, the device may comprise a frame and a fluid impermeable sheath. The sheath may fully enclose the frame and can be temporarily fluid impermeable. The frame may be compressible from an elongated delivery configuration to a compressed squat configuration having a maximum diameter greater that a maximum diameter in the delivery configuration. The frame may have a distal end, a proximal end, and a sidewall section comprising a radially outwardly foldable waist section. The sidewall section may comprise a plurality of elongated struts connecting the distal and proximal ends. The waist may be provided by the elongated struts having an outward inflection zone disposed intermediate the ends of the strut configured to allow the strut fold radially outwardly during deployment. The elongated struts may comprise an inward inflection zone disposed at each end of the strut configured to allow an adjacent section of the strut bend radially outwardly during deployment. The frame may be formed of a bioresorbable material. The frame may be 3-D printed from an extruded material. The frame may be formed from a bioresorbable homopolymer or co-polymer of homopolymers. The distal end may comprise a peripheral flange section that extends radially outwardly of the distal end of the sidewall having a diameter greater than a diameter of the proximal end, for example 1.5, 2.0 or 2.5 times greater. The distal end may be formed from an expansible material such as a firm sponge material. This allows the peripheral flange section of the distal end of the occlusion device to be compressed to fit into the introducer device and then expand radially outwardly when the occlusion device is released from the introducer device. The wide distal end of the deployed occlusion device provides a greater surface area for abutting the adventitial layer of the blood vessel.

As used herein, the term "introducer device" refers to a device configured to deliver an occlusion device into a tissue tract and comprises an ejection module including a distal conduit dimensioned for advancement into the tissue tract and to expose the occlusion device proud an end of the distal conduit. The ejection module may comprise an outer retractable sheath defining the distal conduit configured for axial adjustment from a delivery configuration in which a distal end of the outer retractable sheath extends distally (i.e. providing a protective occlusion device delivery chamber for receipt of the occlusion device) and a deployment configuration where the outer retractable sheath is retracted distally (e.g. to expose the occlusion device to the surrounding wall of the tissue tract.). The introducer device generally also includes a deployment module for the occlusion device, for example a rotation to translation movement actuator having a knob-like rotor, connected to a proximal end of the occlusion device by a deployment tube, whereby the rotor can be rotated to urge the deployment tube towards the occlusion device to push the proximal end of the occlusion device towards the distal end of the occlusion device to deploy the occlusion device. The introducer device may comprise one or more control wire lumens to house control wires. The introducer device can also include a control wire locking module provided with a clamp-like grip movable between a control wire holding or locking position to temporarily maintain/hold the occlusion device in the deployed squat and radially expanded configuration and a control wire release position.

As used herein, the term "control wire" refers to an elongated element that can connect the locking module of the introducer device and the occlusion device. It may be provided by a suture. The control wire is generally bioresorbable. The control wire may be a closed loop that is coupled with the locking module, or a wire with free ends that are coupled to the locking module. Typically, the or each control wire is a suture.

The term "injectable filler material" refers to a material that is injectable through a through lumen in the introducer device and forms a fluid impermeable tamponade (e.g., a plug) in the lumen of the occlusion device to prevent blood flow through the deployed occlusion device. Examples include collagen filler materials and cyanoacrylate glues (Covidien). The filler is generally a bioresorbable material and is adsorbed by the body over time. The filler is generally an expansible material. The filler may be a thermosetting material that is sufficiently fluid at room temperature to be injected into the occlusion device and that hardens or increases in viscosity at body temperature to form a fluid impermeable tamponade in the occlusion device. Other examples of filler materials include hydrogels. Typically, the filler material has a viscosity of 30 cP to 1500 cP measured using a Brookfield viscometer at room temperature and ambient pressure. Typically, the filler material comprises a polymer/monomer such as collagen or cyanoacrylate and an aqueous solvent. Typically, the filler material comprises 3.0mg/ml to 9.9mg/ml polymer (or monomer).

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

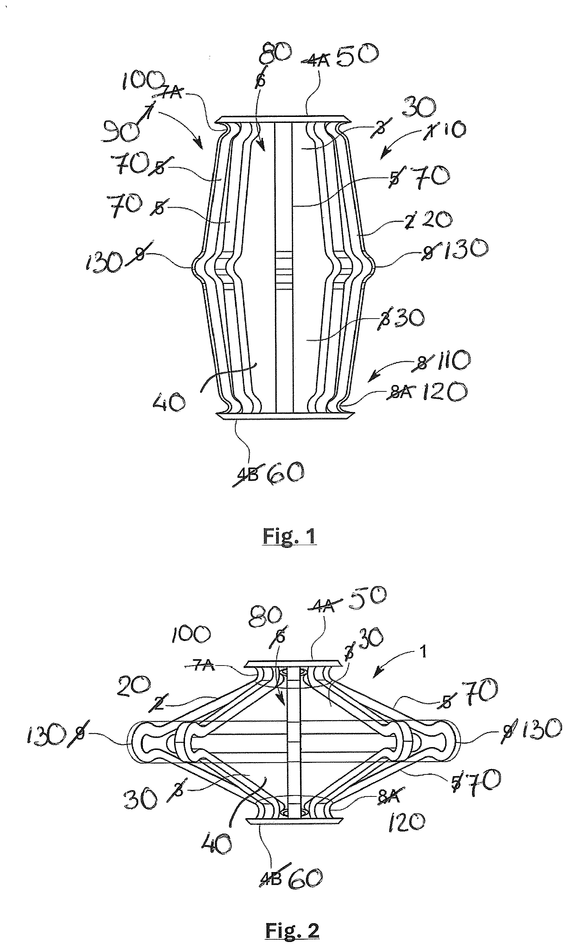

Referring to the drawings, and initially to Figures 1 to 7, there is illustrated an occlusion device indicated generally by the reference numeral 10 which is suitable for use in the system of the invention. The described occlusion device 10 is simply an example of an occlusion device suitable for use in the system of the invention that is radially expansible from a delivery configuration to a radially expanded deployed configuration and it will be appreciated that the invention is not limited to this specific occlusion device and other occlusion devices that are radially expansible from a delivery configuration to a radially expanded deployed configuration can be employed in the system of the invention.

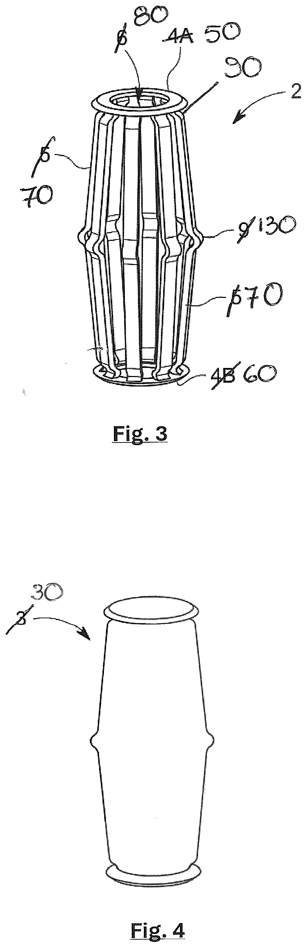

The occlusion device 10 comprises a generally cylindrical frame 20 enclosed within a flexible fluid-impermeable sheath 30. In some embodiments, the device may include a balloon 40 which is dimensioned to fit within the frame 20 and functions to receive and contain an injectable filler material (e.g. collagen) which may be injected into the balloon 40 when the device 10 has been deployed in-vivo and serves to maintain the shape of the deployed device 10 and prevent fluid permeating through the deployed device. Figures 1 and 2 show the assembled occlusion device 10 with the frame 20 disposed inside the sheath 30 and the balloon 40 disposed within the frame 20 and Figures 3 to 7 show the frame 20, sheath 30 and balloon 40 prior to assembly.

The frame 20 comprises a distal end 50 and a proximal end 60 connected by eight elongated struts 70, defining a hollow lumen 80. In this embodiment, the frame 20 is formed from an extruded bioabsorbable co-polymer that is printed into the frame-shape. Each strut 70 has a distal end 90 with an inward inflection zone 100, a proximal end 110 with an inward inflection zone 120, and an outward inflection zone 130 disposed intermediate the distal end 90 and proximal end 110 that is configured to allow the strut fold outwardly during deployment, to form the waist section of the device. The configuration of the struts 70, in particular the inner inflection zones 100,120 at each end of the strut 70 and the outer inflection zone 130 in the middle of the strut 70 , urges the frame 20 to compress into a "Chinese lantern" shape when axial torque is applied to either end of the frame 20 thus compressing the frame 20 from an elongated delivery configuration illustrated in Figure 1 to a compressed (squat) deployed configuration illustrated in Figure 2.

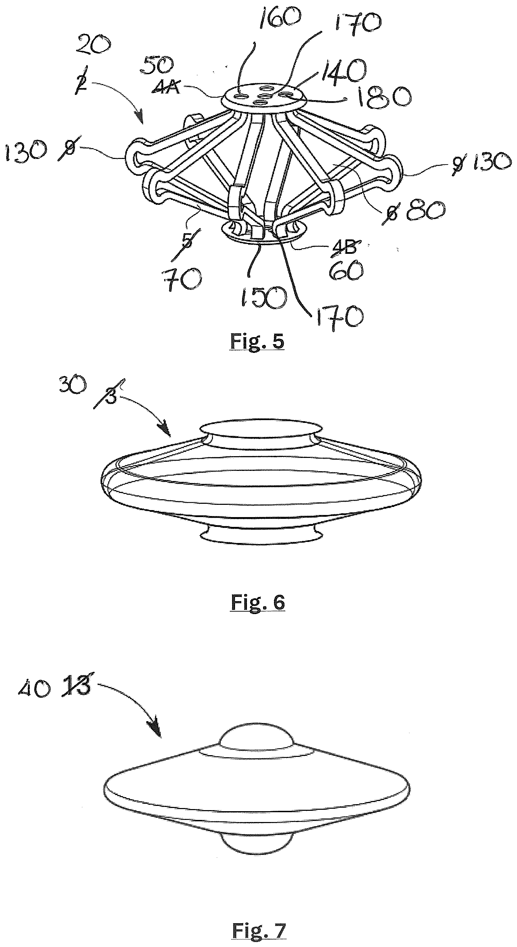

In this embodiment, the flexible sheath 30 and frame 20 are formed from a bioresorbable material (such as Zeus Absorv (Trade Marks), PGLA or a magnesium alloy). The sheath 30 is shaped to encase and closely adhere to the frame 20 in a delivery configuration, as shown in Figure 4, in which the sheath 30 is shown in a relaxed non-tensioned configuration. As the sheath 30 is formed from a flexible material, it can stretch to follow the shape of the frame 20 as it is deployed from the shape shown in Figure 4 to the deployed shape of Figure 6, where the sheath 30 will be slightly tensioned but not enough to alter the shape of the frame 20.

As shown particularly in Figure 5 the distal end 50 and proximal end 60 are provided with a distal plate 140 and a proximal plate 150, respectively. The distal plate 140 has five apertures 160, including a central aperture 170 for receipt of a guidewire and four peripheral apertures 180 (two on each side of the plate 140) for receipt of control wires (not shown). Similarly, the proximal plate 150 also has five apertures 160, including a central aperture 170 for receipt of a guidewire and four peripheral apertures 180 (two on each side of the plate 150) for receipt of control wires (not shown).

As shall be explained more fully below, deployment of the occlusion device 10 may employ one or more control wires that are operably coupled between a control wire locking module of an introducer device and the occlusion device 10. The control wire locking module can co-operate with a deployment module of the introducer device to deploy the occlusion device 10. Generally, at least two control wires are employed, one connected to each side of the occlusion device 10 to balance the torque being applied to the occlusion device 10 and ensure that it deploys evenly. Each control wire may be a closed loop or a wire with free ends. In the embodiments described, the control wire is provided by a bioresorbable suture, but it will be appreciated that it may be provided by any other suitable elongate element. In order to move the occlusion device 10 between the delivery configuration shown in Figure 1 and the squat deployed configuration of Figure 2, the occlusion device 10 can be subjected to a compressive force to urge the distal and proximal ends 50,60 (i.e. the distal and proximal plates 140,150) towards each other.

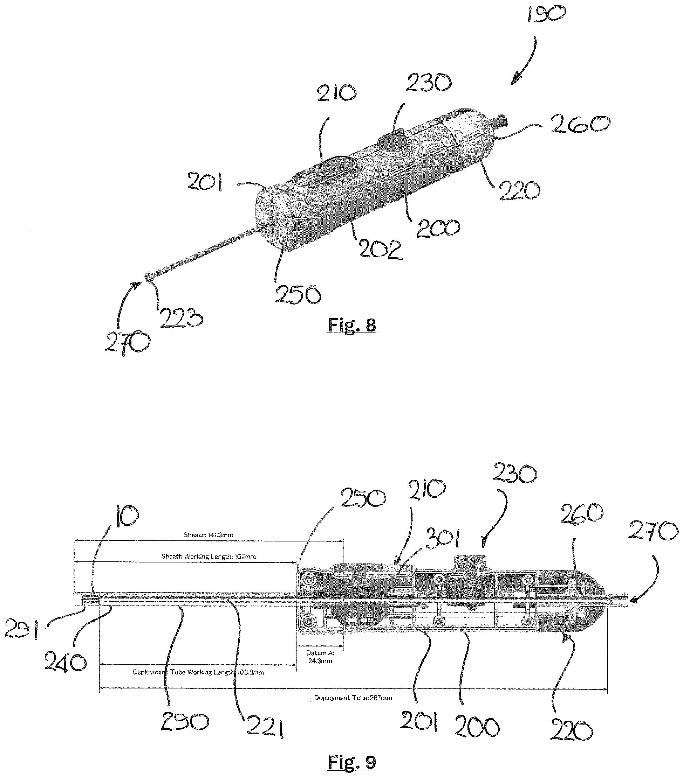

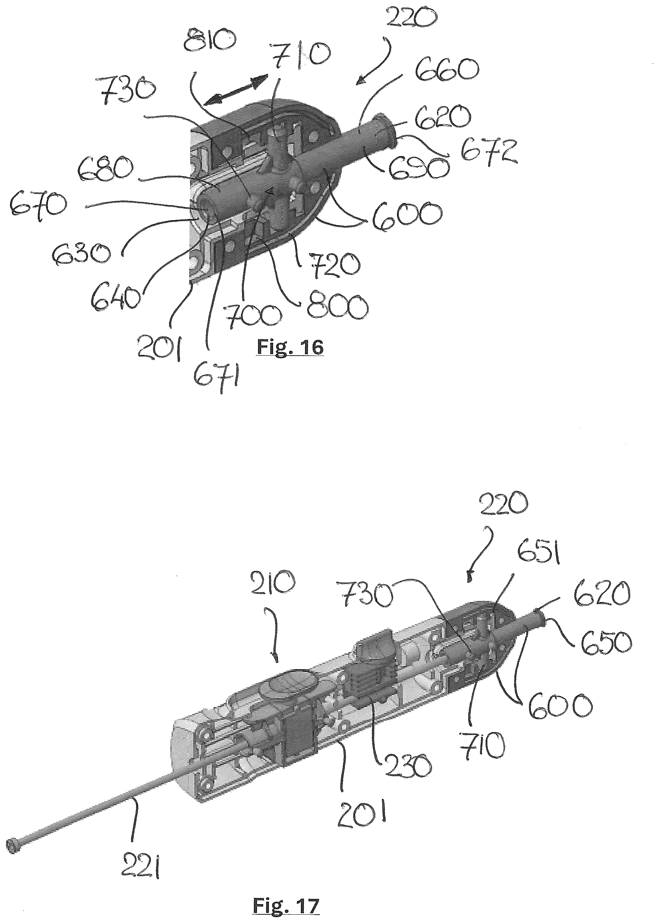

Figures 8 to 21 show an introducer device forming part of a system according to the invention and indicated generally by the reference numeral 190 configured to deliver and deploy an occlusion device 10 such as the occlusion device 10 of Figures 1 to 7. It will be appreciated by those skilled in the art that the introducer device 190 is adapted to deliver and deploy any occlusion device provided the occlusion device is configured to be movable between an elongate delivery configuration and a squat deployed configuration as with the occlusion device 10 of Figures 1 to 7. Accordingly, other occlusion devices which differ structurally to the occlusion device 10 of Figures 1 to 7 can form part of the system of the invention.

Generally, the introducer device 190 comprises a proximal handle 200 provided with an ejector module 210 having an occlusion device delivery conduit 240 extending from a distal end 250 of the handle 200 for ejecting an occlusion device 10 from the introducer device 190, a deployment module 220 to move the occlusion device 10 between an elongate delivery position and a squat radially expanded deployment position and a control wire locking module 230 connected with the occlusion device 10 via control wires 231 to temporarily maintain/hold the occlusion device 10 in the deployed squat and radially expanded configuration during deployment of the occlusion device 10 in a tissue tract.

The introducer device 190 also comprises a through lumen 270 extending through the handle 200 and the delivery conduit 240 for receipt of a guidewire (not shown) and to allow delivery of the injectable filler material into the lumen 80 of a deployed occlusion device 10 to permanently maintain the occlusion device 10 in a squat radially expanded configuration.

In the present embodiment, the ejector module 210 is disposed towards the handle distal end 250, the deployment module 220 is disposed towards the handle proximal end 260 and the locking module 230 is located between the ejector module 210 and the deployment module 220. However, in other embodiments, the relative positions of the modules can be reversed if desired.

As shown particularly in Figures 9, 10, 11 and 12, the ejector module 210 is a slidable ejector module 210 which can be located towards the distal end 250 of the handle 200 (which can be formed in two assembled parts - a right hand side (RHS) 201 and a left hand side (LHS) 202). In the present embodiment, the slidable ejector module 210 is made up of an ejector carriage 280 slidably mounted in the handle 200 and an elongate ejector sheath 290 defining the delivery conduit 240 which extends distally from the ejector carriage 280 through a forward opening 203 defined in the handle 200. The ejector carriage 280 is slidable in oppositely disposed tracks 300 defined in the RHS and LHS 201,202 of the handle 200 between an occlusion device delivery configuration (i.e. the ejector sheath is in an extended position to cover the occlusion device 10) shown in Figure 9 and a retracted deployment position to expose the occlusion device for deployment shown in Figure 10. In the occlusion device delivery configuration (i.e. the ejector sheath is in an extended position) shown in Figure 9, the ejector sheath 290 defines a protective delivery chamber 291 in the delivery conduit 240 for an occlusion device 10. Movement of the slidable carriage 280 between the occlusion device delivery configuration and the retracted deployment position can be effected by a spring-loaded push-button 310 mounted on the slidable carriage 280 at a spring 320.

The slidable carriage 280 is made up of a housing 330 defining a transverse bore 340 for receiving a deployment tube 221 forming part of the deployment module 220 and externally projecting lugs 350 each side of the housing 330 which form a sliding relationship with the carriage tracks 300 defined by the handle 200. The deployment tube 221 extends distally from the proximal end 260 of the handle 200 and terminates at its distal end at an end plate 223. The deployment tube 221 extends proximally to the deployment module at the handle proximal end 260. The end plate 223 is provided with apertures 224 (see also Figures 20 and 21)complementary with the apertures 160 of the occlusion device 10 to receive the guide wires. Internally, the deployment tube 221 defines the through lumen 270 for receiving the guide wire and the injectable filler material in use.

The handle 200 is also provided with a top elongate slot 360 through which the spring-loaded push-button 310 extends so that the push-button 310 can slide with the carriage 280 in use. More particularly, the push-button 310 is made up of an upper finger-depressible head 370 which projects through the elongate slot 360 connected via a neck 371 to a lower body 380 which can be pushed downwards against the bias of the spring 320. A stop member 301 is provided at each end of the carriage tracks 300 which is abuttable with the neck 371 to prevent further travel of the sliding carriage 280 in use.

The push-button 310 together with the handle 200 also define an ejector module catch 385 at each end of the carriage tracks 300 between the stop members 301 to hold the ejector module 210 in the occlusion device delivery configuration and the retracted deployment position. In the present embodiment, the ejector module catch 385 is made up of a catch member 390 either side of the push-button body 380 engageable with complementary recesses 400 defined in the handle 200 towards each end of the ejector carriage tracks 300.

Accordingly, upon depression of the push-button head 370 against the bias of the spring 320 the catch members 390 are urged from their respective complementary recesses 400 so that the slidable carriage can be moved as required.

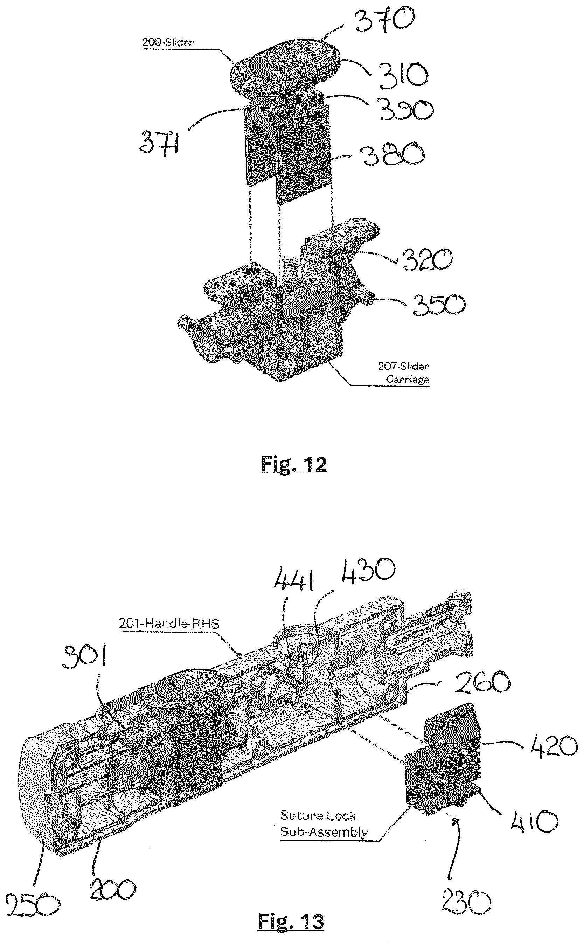

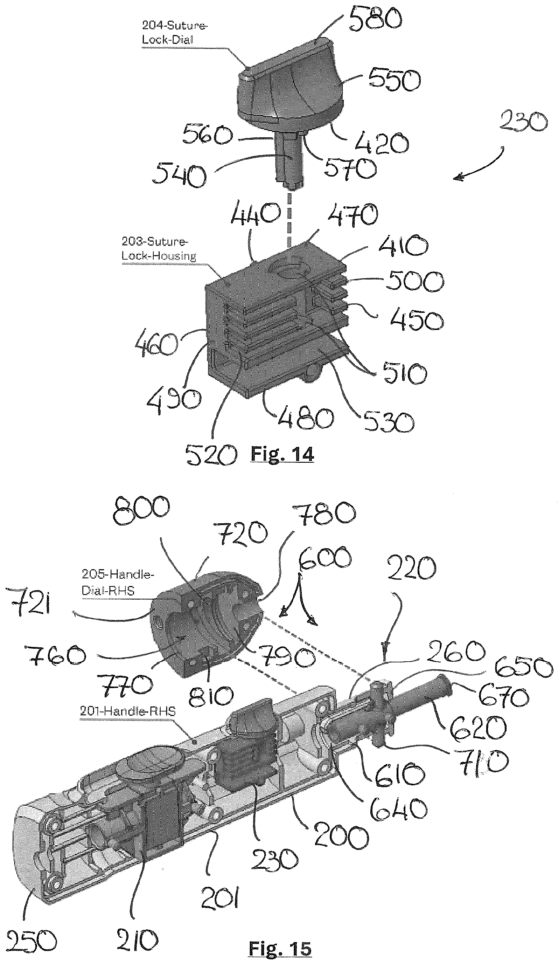

Figure 13 shows an enlarged partially exploded and cross-sectional view through the handle 200 of the introducer device of Figure 11 with the ejector module 210 in place in the handle 200 in the occlusion device delivery configuration and the control wire locking module 230 detached from the handle 200 for clarity and Figure 14 shows an exploded view of the control wire locking module 230 detached from the handle 200 for clarity. As shown in the drawings, the control wire locking module 230 can be a two-part locking module 230 made up of a housing which functions as a lock 410 and a dial-like controller which can be in the form of a key 420 which is complementary with the lock 410 and is movable between a control wire holding/clamping position and a control wire release position. The locking module 230 can be mounted in the handle 200 at a locking module mounting 430 located between the ejector module 210 and the deployment module 220 via one or more mounting pins 440 provided on the lock 410 insertable in corresponding holes defined in the handle 200.

The lock 410 is generally boxlike in shape and is made up of a front face 450, a rear face 460 provided with the outwardly projecting mounting pins 440, a top face 470, a bottom face 480, a distal side face 490 and a proximal side face 500. A keyed through hole 510 defining a keyway 510, which can be partially defined by the rear face 460, for receiving the key 420 extends from the top face 470 towards the bottom face 480. The front face 450 is shaped and contoured to define transverse control wire guide channels 520 for receiving and guiding control wires 231 from the occlusion device 10 through the introducer device 190. The lock 410 is further provided with a transverse deployment tube guide channel 530 which can be beneath the control wire guide channels 520 for guiding and supporting the deployment tube 221 through the handle 200.

The key 420 is made up of a key shaft 540 insertable in the complementary keyway 510 and a key head 550 which can be dial-like in configuration and be provided with a finger-grip 580. The key shaft 540 is provided with a biting 560 for gripping or clamping the control wire between the biting and the keyway 510 by forming an interference fit in the keyway 510 when the key 420 is in the control wire holding/clamping position. In this embodiment, the biting 560 can be in the form of a gripping flange 560 which extends laterally outwards from the key shaft 540 to abut the rear face 460 in the keyway 510 in an interference fit in the control wire holding/clamping position to lock or hold a control wire in place between the biting 560 and the keyway 510.

The key shaft 540 can also be provided with a shoulder stop 570 adjacent the key head 550 to prevent over-rotation of the key 420 in the keyway 510.

Figures 15 to 19 show the handle of the introducer device 190 with the ejector module 210 and the control wire locking module 230 in place in the handle 200 together with the deployment module 220. As shown particularly in Figures 15 to 17, the deployment module 220 is made up of a manually operable actuator 590 which can be a rotation to translation movement actuator 600 provided at a proximal neck 610 of the handle 200. The rotation to translation movement actuator 600 can be a two-part actuator made up of an axially slidable or translating rod 620 mounted in a cavity 630 in the handle neck 610 sized and shaped to receive the translating rod 620, and a rotor 720. In the present embodiment, the cavity 630 extends between an internal distal opening 640 in the handle neck 610 and a proximal exit opening 650 in the handle neck 610, which is surrounded by an outwardly projecting rim 651, through which the translating rod 620 can project from the introducer device 190.

The translating rod 620 is formed by an elongate tubular shaft 660 which is open at both ends defining an internal through duct 670 having an inner opening 671 and outer opening 672 for receiving the deployment tube 221. More particularly, the internal duct 670 is made up of a distal deployment tube receiving portion 680 and a proximally contiguous handle exiting portion 690 with an internal stop 700 between the two portions 680, 690 which abuts the proximal end of the deployment tube 221. The internal duct 670 is therefore contiguous with the through lumen 270 of the deployment tube 221. Externally, the tubular shaft 660 is provided with two oppositely disposed laterally extending male fingers 710 which are rotatably engageable with the rotor 720 which in the present embodiment is manually operable. The tubular shaft 660 is further provided with outwardly projecting oppositely disposed pairs of male lugs 730 which are slidable in complementary deployment tracks 740 defined in the handle neck 610 in the RHS 201 and LHS 202 of the handle 200 at the handle neck 610. The handle neck 610 is also provided with upper and lower finger slots 750 (see also Figure 11) through which the male fingers 710 can project from the handle neck 610 to engage the rotor 720.

The rotor 720 is made up of a body 721, which in the present embodiment is knob-like in shape but can have other shapes as required, having a RHS and LHS for assembly purposes with the RHS shown in the drawings. Internally, the body 721 defines a neck chamber 760 shaped and contoured to receive the handle neck 610. More particularly, the neck chamber 760 extends between a larger distal neck opening 770 for encircling the handle neck 610 and a proximal smaller translating rod opening 780 for encircling the translating rod 620. The body 721 is provided with a continuous recess (when the RHS and LHS of the body 721 are assembled) 790 for receiving the rim 651 at the proximal exit opening 650 of the neck 610 and to enable the rotor 720 to rotate about the rim 651. Internally, the body 721 is also threaded to receive the male fingers 710 of the translating rod 620 in a rotating relationship. The thread(s) 800 are configured to limit rotation of the rotor 720 on the neck 610 to 180° between the occlusion device elongate delivery position of Figure 1 and the occlusion device squat radially expanded deployment position of Figure 2. In the present embodiment, the body 721 is provided with two separate oppositely disposed threads or grooves 800 - (one in the RHS and one in the LHS of the rotor 720) for receiving the male fingers 710 to limit the rotation to 180° between an occlusion device elongate delivery position and an occlusion device squat radially expanded deployment position with each thread 800 being provided with thread stops 810 at each end to limit further movement of the male fingers 710 in the threads 800. In other embodiments of the invention, other thread arrangements and configurations are possible.

Rotation of the rotor 720 therefore results in axial movement and more particularly distal and proximal translational movement of the translating rod 620 to in turn effect axial and more particularly distal and proximal translational movement of the deployment tube 221 towards and away from the occlusion device 10. As shown in Figures 20 and 21, distal translational movement of the deployment tube 221 towards the occlusion device 10 exerts a distal compressive force on the occlusion device 10 to move the occlusion device 10 between the elongate delivery configuration and the deployed squat radially expanded position. Conversely, proximal translational movement of the deployment tube 221 removes the distal compressive force and allows the occlusion device 10 to return to the elongate delivery configuration if required. The occlusion device 10 can return to the elongate delivery configuration due to a restoring force in the occlusion device 10 e.g. a spring-like force due to the structure of the occlusion device 10 and/or the resilience and/or elasticity of the materials of the occlusion device 10.

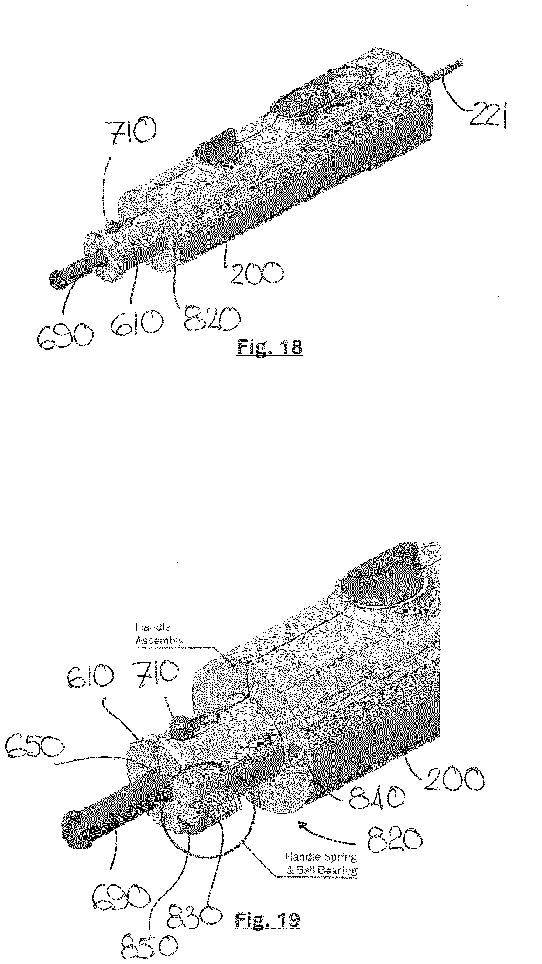

The handle 200 is also provided with a movement controller 820 to achieve controlled rotation of the rotor 720 on the handle neck 610. The movement controller 820 can be a biasing means to ensure a user can accurately and finely move the rotor between the occlusion device elongate delivery and squat radially expanded deployment positions. In the present embodiment, the biasing means can be a spring biased between the handle 200 and the rotor 720. More particularly, as shown in Figure 19, a spring 830 can be inserted in a spring hole 840 defined in the handle 200 adjacent the neck 610 to be biased against the rotor 720 via a bearing, such as a ball bearing 850 disposed in the spring hole 830 between the spring 830 and the rotor 720.

In use, the extra-vascular closure system of the invention is made up of an occlusion device that is radially expansible from an elongate delivery configuration to a radially expanded deployed configuration to occlude a lumen such as a tissue tract, such as the occlusion device 10 of Figures 1 to 7, and the introducer device 190 of Figures 8 to 21 can be operated as follows. Firstly, the introducer device 190 can be fitted with an occlusion device 10 in the ejector module 210 i.e. in the delivery chamber 291 at the deployment tube 221 as previously described. The occlusion device 10 can be held in the delivery chamber 291, in the elongated delivery configuration, via (optionally two) control wires 231 extending from the occlusion device to the locking module 230 via the apertures 224 in the end plate 223 of the deployment tube 221, the delivery conduit 240, the bore 340 of the ejector module 210 and the wire channels 520 in the locking module 230. The control wires 231 therefore pass through the lumen 80 of the occlusion device 10. The occlusion device 10 is protected by the ejector sheath 290 whilst in the delivery chamber 291 for delivery. The locking module 230 is in the control wire release position and the deployment module 220 in the occlusion device elongate delivery position (i.e. the control wires are untensioned).

The introducer device 190 with the occlusion device 10 as shown in Figure 9 is then placed over a guide wire (not shown) via the through lumen 270 in the deployment tube 221 and directed towards a patient's vasculature to eject the occlusion device 10 as required.

The occlusion device 10 is then ejected by depressing the spring loaded push-button 310 and proximally retracting the ejector sheath 290 to reveal the occlusion device 10 from within the protective delivery chamber 291.

The locking module 230 is then actuated to clamp and tension the control wires 231 between the keyway 510 and the key shaft 540. The occlusion device 10 is then deployed into the squat deployed configuration shown in Figure 2 by rotating the rotor 720 on the handle 200 of the introducer device by 180° to distally and axially translate the translating rod 620 and hence the deployment tube 221 distally to compress the occlusion device with a distal axial force 10 against into the occlusion device squat radially expanded deployment position of Figure 2 against the tensioned control wires 231 . In this configuration, the outer inflexion zone 130 extends radially outwardly to embed in the wall of a tissue tract to anchor the occlusion device 10 in the tissue tract. The ejector module 210 and the locking module 230 therefore co-operate to temporarily maintain/hold the occlusion device 10 in the deployed squat and radially expanded configuration

A user can determine by tactile feedback from the deployment module 220 how much the occlusion device 10 has been deployed i.e. the degree of radial expansion of the occlusion device 10. Alternatively, or in addition, a graduated scale on the rotor 720 may also serve to indicate the degree of deployment. Although not illustrated, the rotor 720 may include a graduated scale of French gauge size (Fr) disposed circumferentially on a surface of the rotor 720. An example would an indication of 8 F at 0 degrees (elongated delivery configuration), 12 F at 90°, 16 F at 180° etc. to indicate the diameter of the expanded occlusion device 10 in the squat radially expanded deployment position to oversize the diameter of a tissue tract as required.

The injectable filler material can then be injected into the occlusion device 10 via the outer opening 672 and internal duct 670 of the translating rod 620 of the deployment module 220. The injectable filler material sets the occlusion device in the squat deployed configuration. The locking module 230 is then operated to unclamp the control wires 231 and the control wires 231 can then be severed from the occlusion device 10 and the introducer device 10 removed.

The control wires 231 can be severed manually e.g. with a scissors or, in another embodiment, the introducer device 10 can be provided with a control wire cutting module.

As indicated above, the occlusion device 10 can also be returned to the elongated delivery configuration by reversing the above process prior to injection of the filler material. This may be performed if the initial deployment is not successful, for example if it does not embed properly in the tissue tract or does not fully occlude the tissue tract, allowing the occlusion device 10 to be re-sheathed or moved axially to a different position in the tissue tract.

In use, a typical surgical procedure can comprise the following steps:
1- A vessel/femoral artery is punctured using a small needle US Guided - (micro puncture or Kimmel needle);
2- A soft J tip wire is introduced through the needle then a 6F sheath introduced over the wire;
3- A wire is then reintroduced into the artery and a 9 or 10 F sheath then the large bore sheath is used to deliver a valve or other large intra vascular device . Anything between 14 to 24 Fr sheath can be used depending on procedure. Most cases are 14 to 18 Fr sheath.
4- Large sheath is removed, and +/- contralateral balloon is inflated proximal to sheath entrance hole (Iliac vessel);
5- A large bore vascular closure system is employed by inserting an introducer device 190 fitted with an occlusion device 10 over the guidewire, ejecting the occlusion device 10, deploying the occlusion device 10 to the squat deployed configuration as outlined above, and locking the occlusion device in the squat deployed configuration with the locking module;
6- A syringe for filler material is attached to the proximal end of the introducer device and a syringe needle is advanced through the through lumen 270 of the introducer device 190 into the deployed occlusion device 10 and filler material is injected into the occlusion device 10 where the material serves to maintain the squat deployed configuration of the deployed occlusion device 10 and in time also partially seeps through the walls of the occlusion device 10 to adhere to the walls of the tissue tract anchoring the occlusion device in position.
7- The +/- balloon is deflated. Ultrasound/Fluro Check device positioning and adherence is performed;
8- When haemostasis is achieved, the control wires are severed/removed as outlined above and external dressing applied.

## Claims

1. A large bore extra-vascular closure system to occlude a large bore tissue tract proximal to a blood vessel, comprising:
an introducer device (190) having
a handle (200) housing an ejector module (210) provided with a delivery conduit (240) extending distally from the handle (200) for delivering an occlusion device (10) to a tissue tract;
an occlusion device deployment module (220) provided with a deployment tube (221) extending distally from the handle (200) within the delivery conduit (240) for deploying the occlusion device (10), and
a bioresorbable occlusion device (10) having a distal end (50) and a proximal end (60) configured for adjustment by the deployment module (220) between an elongate delivery configuration in which the occlusion device (10) is dimensioned to fit within the delivery conduit (240) and a squat deployed configuration in which the occlusion device (10) is expanded radially outwardly,
wherein the deployment tube (221) is configured upon actuation to push the proximal end (60) of the occlusion device (10) towards the distal end (50) of the occlusion device (10) to expand the occlusion device (10) radially outwardly into the squat deployed configuration.

2. . A large bore extra-vascular closure system according to Claim 1 wherein the introducer device (190) comprises a control wire operably connecting the distal end (50) of the occlusion device (10) to a control wire locking module (230) co-operable with the deployment module (220) to hold the occlusion device (10) in the squat deployed configuration and an injectable filler material configured for injection into the occlusion device (10) in the squat deployed configuration.

3. A large bore extra-vascular closure system according to Claim 2 wherein the control wire locking module (230) comprises a lock (410) and a key (420) complementary with the lock (410) in which the key (420) is movable between a control wire holding position in which the occlusion device (10) is held in the squat deployed configuration and a control wire release position.

4. A large bore extra-vascular closure system according to Claim 3 wherein the lock (410) comprises a keyway (510) for receiving the key (420) and the key (420) comprises a key shaft (540) with a biting (560) for holding the control wire between the biting (560) and the keyway (510) in the control wire holding position.

5. A large bore extra-vascular closure system according to Claim 4 wherein the biting (560) is configured to define an interference fit with the keyway (560) in the control wire holding position.

6. A large bore extra-vascular closure system according to any of Claims 2 to 5 wherein the locking module (230) comprises a control wire guide channel (520) and/or a deployment tube guide channel (530) configured to support the deployment tube (221) in the handle (200).

7. A large bore extra-vascular closure system according to any of Claims 1 to 6 wherein the ejector module (210) comprises a slidable ejector carriage (280) slidably mounted in the handle (200) and an elongate ejector sheath (290) attached to the ejector carriage (280) defining the delivery conduit (240), the ejector carriage (280) being slidable between an occlusion device (10) delivery configuration in which the ejector sheath (290) is in an extended position over the occlusion device (10) and a retracted occlusion device (10) deployment position in which the occlusion device (10) is exposed for deployment by the deployment module (220).

8. A large bore extra-vascular closure system according to Claim 7 wherein the ejector carriage (280) comprises a housing (330) slidable in a carriage track (300) in the handle (200).

9. A large bore extra-vascular closure system according to Claim 8 wherein the housing (330) comprises a bore (340) for receiving the deployment tube (221).

10. A large bore extra-vascular closure system according to Claim 8 or Claim 9 wherein the ejector module (210) comprises a push-button (310) mounted on the ejector carriage (280) to effect movement of the ejector carriage (280) between the occlusion device (10) delivery configuration and the occlusion device (10) deployment position.

11. A large bore extra-vascular closure system according to any of Claims 8 to 10 wherein the ejector module (210) comprises an ejector module catch (385) towards the end of the carriage track (300) to hold the ejector module (210) in the occlusion device (10) delivery configuration and the occlusion device (10) deployment position.

12. A large bore extra-vascular closure system according to any of Claims 1 to 11 wherein the deployment module (220) comprises a rotation to translation movement actuator (600).

13. A large bore extra-vascular closure system according to Claim 12 wherein the rotation to translation movement actuator (600) comprises a translatable rod (620) in the handle (200) connected with a rotor (720) towards a proximal end (260) of the handle (200).

14. A large bore extra-vascular closure system according to Claim 13 wherein the translatable rod (620) comprises a tubular shaft (660) having laterally extending male fingers (710) and the rotor (720) comprises an internally threaded body (721) configured to receive the male fingers (710) of the translating rod (620) in a rotating relationship.

15. A large bore extra-vascular closure system according to Claim 14 wherein the body (721) comprises two oppositely disposed threads (800) configured to receive two respective male fingers (710) and to limit rotation of the rotor (720) to about 180° between an occlusion device (10) elongate delivery position and an occlusion device (10) squat radially expanded deployment position.
